# EUROPEAN PATENT APPLICATION

(11) **EP 3 747 470 A1**
(43) Date of publication of application: **09.12.2020**
(21) Application number: 19746702.0
(22) Date of filing: 04.02.2019
(51) Int. Cl.: A61K 45/00, A61K 31/7088, A61K 31/713, A61K 39/395, A61K 48/00, A61P 17/00, A61P 17/02, A61P 17/06, A61P 17/14, A61P 29/00, A61P 37/00, C12Q 1/6851, C07K 16/40, C12N 15/113

(54) **DRUG FOR CHRONIC INFLAMMATORY DISEASE AND METHOD FOR EVALUATING SUSCEPTIBILITY TO CHRONIC INFLAMMATORY DISEASE**

(30) Priority: 02.02.2018 JP 2018016930
(71) Applicant: National University Corporation Hokkaido University, Sapporo-shi, Hokkaido 060-0808 (JP)
(72) Inventor: MURAKAMI, Masaaki, Sapporo-shi, Hokkaido 060-0808 (JP); KAMIMURA, Daisuke, Sapporo-shi, Hokkaido 060-0808 (JP); FUJITA, Munezumi, Sapporo-shi, Hokkaido 060-0808 (JP)
(74) Representative: Wasner, Marita
(86) International application number: PCT/JP2019/003926
(87) International publication number: WO 2019/151524

(57) **Abstract**

[Problem] The purpose of the present invention is to provide new treatment targets and treatment methods for chronic inflammatory disease based on a molecular biological mechanism relating to the onset of keloid. [Solution] The present invention relates to a drug for the prevention and/or treatment of chronic inflammatory disease that contains as an active ingredient a substance that suppresses the expression and/or inhibits the activity of a specific NEDD4 splicing variant protein. Also provided are a method for evaluating the susceptibility of a subject to chronic inflammatory disease and a method for evaluating the suitability of treatment of a chronic inflammatory disease by the abovementioned drug that include a step for detecting the NEDD4 splicing variant protein or mRNA in a collected tissue, a drug for preventing and/or treating chronic inflammatory disease that contains as an active ingredient a substance that suppresses the expression and/or inhibits the activity of NEDD4 protein, and a method for evaluating the susceptibility of a subject to chronic inflammatory disease that includes a step for detecting a specific SNP of NEDD4.

## Description

### Field

The present invention relates to a pharmaceutical for preventing and/or treating a chronic inflammatory disease in which the pharmaceutical contains a substance for suppressing the expression and/or inhibiting the activity of NEDD4 splicing variant protein as an active ingredient; a method for determining susceptibility to a chronic inflammatory disease in which the method uses the expression level of NEDD4 splicing variant protein as an index; and a method for determining suitability for treatment with the pharmaceutical described above for a chronic inflammatory disease. The present invention also relates to a pharmaceutical for preventing and/or treating a chronic inflammatory disease in which the pharmaceutical contains a substance for suppressing the expression and/or inhibiting the activity of NEDD4 protein as an active ingredient; and a method for determining susceptibility to a chronic inflammatory disease in which the method uses a SNP in NEDD4 gene as an index.

### Background

Keloid is a chronic inflammatory skin disease in which fibrous components in a scar proliferate excessively after injury, surgery, or the like, and which involves immune cells. The characteristic histological findings of keloids are mainly dermal fibroblast proliferation, and irregular overproduction of collagen fibers as an extracellular matrix. Keloids are categorized as benign fibroproliferative lesions. However, keloid is one of the diseases that significantly impair the quality of life (QOL) in patients. A reddish-brown, raised, firm mass may be a cosmetic nuisance. It is often accompanied by persistent itching and pain. In particular, when the mass has a complicated shape or a roughened surface, deep infection can cause recurrent pus discharge, for example.

NEDD4 (neural precursor cell expressed developmentally down-regulated protein 4, E3 ubiquitin protein ligase; may also be referred to as NEDD4-1) is known as one of keloid susceptibility genes found by performing genomic analysis on Japanese patients with keloids (Non Patent Literature 1). The protein encoded by the NEDD4 gene is a ubiquitin protein ligase, and is known to be involved in controlling membrane protein, transmitting signals, decomposing protein, and the like.

As for the relation between keloids and NEDD4, in addition to the report by Nakashima et al. described above, there is a report stating that NEDD4 enhances the proliferative capacity and invasiveness of fibroblasts as well as the expression of fibronectin and type I collagen, and is involved in excessive accumulation of an extracellular matrix (Non Patent Literature 2). Consequently, it is considered that the increase in NEDD4 expression may be involved in keloid formation. Moreover, as for the relation between NEDD4 and inflammatory diseases, there is a report stating that in mice co-administered with NEDD4 gene, HIV gag gene, and env gene, the gag responsive IL-6 expression is enhanced in the spleen cells (Non Patent Literature 3). In the present specification, the term "NEDD4" refers to NEDD4 gene, NEDD4 protein that is a ubiquitin ligase encoded by the NEDD4 gene, or refers to both. Unless otherwise specified, the meaning of the term is defined by its context.

Treatment of keloids includes treatment with drugs such as topical therapy (for example, application of steroid ointment or cream), internal therapy (for example, tranilast), and local steroid injection therapy. In addition, cryotherapy, surgical therapy, radiation therapy, and the like may be added or combined. However, a molecular biological mechanism relating to the excessive proliferation of fibrous components triggered by injury remains unknown. Therefore, effective treatment with fewer side effects such as recurrence is not yet established.

Moreover, the development of an effective pharmaceutical has still been desired for preventing and/or treating other chronic inflammatory skin diseases such as hypertrophic scar, psoriasis, and pressure ulcer that are lesions related to keloid, as well as a chronic inflammatory disease generated in a site different from skin such as arthritis.

### Citation List

### Non Patent Literature

Non Patent Literature 1: Nakashima et al., Nat. Genet., 2010, 42, 768-771.
Non Patent Literature 2: Chung et al., Proc. Jpn. Acad. Ser. B 2011, 87, 563-73.
Non Patent Literature 3: Lewis et al., PloS One. 2014, 9(3), e91267.

### Summary

### Technical Problem

An object of the present invention is to provide new treatment targets and treatment methods for chronic inflammatory disease, on the basis of a molecular biological mechanism relating to the onset of keloid.

### Solution to Problem

The present inventors have found out that the expression of a specific NEDD4 splicing variant (may also be referred to as a transcript variant) is enhanced in skin tissue of keloid patients, and that the splicing variant is positively controlling the inflammation amplifier involved with IL-6 and NF-κB, and have completed the following inventions.
(1) A pharmaceutical for preventing and/or treating a chronic inflammatory disease that develops in skin, tendon, or tendon-surrounding tissue, the pharmaceutical containing: a substance for suppressing expression and/or inhibiting activity of NEDD4 splicing variant 3 (SP3) protein as an active ingredient.
(2) The pharmaceutical according to (1) above, wherein the substance for suppressing the expression of the protein is an inhibitory nucleic acid that suppresses expression of SP3 mRNA or that inhibits translation of the protein from the mRNA.
(3) The pharmaceutical according to (1) above, wherein the substance for inhibiting the activity of the protein is an antibody or a derivative thereof that specifically binds to the protein.
(4) The pharmaceutical according to any one of (1) to (3) above, wherein the chronic inflammatory disease includes keloid, hypertrophic scar, psoriasis, atopic dermatitis, bullous pemphigoid, scleroderma, or Dupuytren's contracture.
(5) A method for determining susceptibility of a subject to a chronic inflammatory disease that develops in skin, tendon, or tendon-surrounding tissue, the method including: a step of measuring an expression level of SP3 protein or SP3 mRNA in a sample collected from a subject; a step of comparing the measured expression level with an expression level of SP3 protein or SP3 mRNA in a sample collected from a healthy person; and a step of determining that the subject is susceptible to the chronic inflammatory disease, when the expression level in the sample from the subject is greater than that in the sample from the healthy person.
(6) The method according to (5) above, wherein the chronic inflammatory disease includes keloid, hypertrophic scar, psoriasis, atopic dermatitis, bullous pemphigoid, scleroderma, or Dupuytren's contracture.
(7) A method for determining suitability for treatment with a pharmaceutical for a chronic inflammatory disease that develops in skin, tendon, or tendon-surrounding tissue, the pharmaceutical containing: a substance for suppressing expression and/or inhibiting activity of SP3 protein as an active ingredient, and the method including: a step of measuring an expression level of SP3 protein or SP3 mRNA in a sample collected from a subject; a step of comparing the measured expression level with an expression level of SP3 protein or SP3 mRNA in a sample collected from a healthy person; and a step of determining that the treatment with the pharmaceutical is suitable for the chronic inflammatory disease in the subject, when the expression level in the sample from the subject is greater than that in the sample from the healthy person.
(8) The method according to (7) above, wherein the chronic inflammatory disease includes keloid, hypertrophic scar, psoriasis, atopic dermatitis, bullous pemphigoid, scleroderma, or Dupuytren's contracture.
(9) A pharmaceutical for preventing and/or treating a chronic inflammatory disease (excluding keloid), the pharmaceutical containing: a substance for suppressing expression and/or inhibiting activity of NEDD4 protein as an active ingredient.
(10) The pharmaceutical according to (9) above, wherein the substance for suppressing the expression of the protein is an inhibitory nucleic acid that suppresses expression of mRNA encoding the protein or that inhibits translation of the protein from the mRNA.
(11) The pharmaceutical according to (9) above, wherein the substance for inhibiting the activity of the protein is an antibody or a derivative thereof that specifically binds to the protein.
(12) The pharmaceutical according to any one of (9) to (11) above, wherein the chronic inflammatory disease includes arthritis, hypertrophic scar, psoriasis, atopic dermatitis, bullous pemphigoid, scleroderma, or Dupuytren's contracture.
(13) A method for determining susceptibility of a subject to a chronic inflammatory disease (excluding keloid), the method including: a step of detecting, in a sample collected from a subject, rs8032158 that is a gene polymorphism of a base at position 26 in a base sequence represented by SEQ ID No. 43 present in a genomic sequence encoding an NEDD4 gene; and a step of determining that the subject is susceptible to the chronic inflammatory disease (excluding keloid), when a genotype of the gene polymorphism is C/C or C/T.
(14) The method according to (13) above, wherein the chronic inflammatory disease includes hypertrophic scar, psoriasis, atopic dermatitis, bullous pemphigoid, scleroderma, or Dupuytren's contracture.
(15) A kit for determining susceptibility to a chronic inflammatory disease that develops in skin, tendon, or tendon-surrounding tissue, the kit including: a primer set or a probe that detects expression of SP3 mRNA.
(16) A kit for determining susceptibility to a chronic inflammatory disease that develops in skin, tendon, or tendon-surrounding tissue, the kit including: a specific antibody against SP3 protein.
(17) A kit for determining suitability for treatment with a pharmaceutical for a chronic inflammatory disease that develops in skin, tendon, or tendon-surrounding tissue, the kit including: a primer set or a probe that detects expression of SP3 mRNA, wherein the pharmaceutical contains a substance for suppressing expression and/or inhibiting activity of SP3 protein as an active ingredient.
(18) A kit for determining suitability for treatment with a pharmaceutical for a chronic inflammatory disease that develops in skin, tendon, or tendon-surrounding tissue, the kit including: a specific antibody against SP3 protein, wherein the pharmaceutical contains a substance for suppressing expression and/or inhibiting activity of SP3 protein as an active ingredient.
(19) A kit for determining susceptibility to a chronic inflammatory disease (excluding keloid), the kit including: a primer set or a probe that detects rs8032158 that is a gene polymorphism of a base at position 26 in a base sequence represented by SEQ ID No. 43 present in a genomic sequence encoding an NEDD4 gene.

### Advantageous Effects of Invention

With the present invention, it is possible to provide a new pharmaceutical that is effective and that has fewer side effects such as recurrence for preventing and/or treating keloid or a chronic inflammatory disease including the keloid, on the basis of a molecular biological mechanism relating to the onset of keloid. Moreover, a method and a kit for determining susceptibility of a subject to a chronic inflammatory disease are also provided. Consequently, it is possible to prophylactically administer the pharmaceutical described above to a subject who is proved to be susceptible to a chronic inflammatory disease prior to the onset of the disease, and effectively prevent the onset of chronic inflammatory disease. Moreover, a method and a kit for determining suitability for treatment with the pharmaceutical described above for a chronic inflammatory disease are also provided. By using the method and kit as companion diagnostics, it is possible to effectively and efficiently use the pharmaceutical described above.

### Brief Description of Drawings

Fig. 1 is a graph illustrating relative expression levels of NEDD4 splicing variants (SP1 to SP7) mRNAs in keratinocytes derived from normal and keloid skin tissues;
Fig. 2 is images of normal and keloid skin tissues immunostained with anti-SP3 protein antibody;
Fig. 3 is graphs illustrating IL-6 promoter activity and NF-κB promoter activity under TNFα stimulation in HEK293T cells in which SP1 or SP3 is forcibly expressed;
Fig. 4 is graphs illustrating relative expression levels of IL-6 mRNA and SP3 mRNA under IL-6 and TNFα stimulation in H4 cells introduced with siRNA for suppressing SP3 expression;
Fig. 5 is western blot images illustrating phosphorylation of NF-κB p65 subunit under TNFα stimulation in H4 cells introduced with siRNA for suppressing SP3 expression;
Fig. 6 is graphs illustrating relative expression levels of IL-6 mRNA and NEDD4 mRNA under IL-6 and IL-17 stimulations in HaCat cells introduced with siRNA (si5-NEDD4, si6-NEDD4, si7-NEDD4) for suppressing the expression of all NEDD4 splicing variants;
Fig. 7 is graphs illustrating relative expression levels of IL-6 mRNA and NEDD4 mRNA under IL-6 and IL-17 stimulations in H4 cells introduced with siRNA (si5-NEDD4, si6-NEDD4, si7-NEDD4) for suppressing the expression of all NEDD4 splicing variants;
Fig. 8 is a graph illustrating changes in clinical scores in rheumatoid arthritis model animals administered with shRNA (NEDD4 sh-1, NEDD4 sh-2) for suppressing the expression of all NEDD4 splicing variants;
Fig. 9 is a graph illustrating changes in the ear thickness of dermatitis model animals administered with siRNA for suppressing the expression of all NEDD4 splicing variants;
Fig. 10 is a graph illustrating the SNP frequency of NEDD4 genes in patients with chronic inflammatory skin diseases; and
Fig. 11 is a graph illustrating the SNP frequency of NEDD4 genes in patients with chronic inflammatory skin diseases and a disease in tendon-surrounding tissue.

### Description of Embodiments

A first aspect of the present invention relates to a pharmaceutical for preventing and/or treating a chronic inflammatory disease that develops in skin, tendon, or tendon-surrounding tissue, the pharmaceutical containing a substance for suppressing the expression and/or inhibiting the activity of SP3 protein.

Seven types of splicing variants SP1 to SP7 are present in human NEDD4, and six types of them excluding SP6 encode protein. The base sequence of NEDD4 gene in the human genome is registered in National Center for Biotechnology Information (NCBI) (https://www.ncbi.nlm.nih.gov/) as NG_051072.1. Moreover, the mRNA base sequences of SP1, SP2, SP3, SP4, SP5, and SP7 are registered in NCBI (https://www.ncbi.nlm.nih.gov/) as NM_006154, NM_198400, NM_001284338, NM_001284339, NM_001284340, and NM_001329212, respectively. The amino acid sequences thereof are registered in NCBI (https://www.ncbi.nlm.nih.gov/) as NP_006145.2, NP_940682.2, NP_001271267.1, NP_001271268.1, NP_001271269.1, and NP_001316141, respectively. In the present invention, in addition to the NEDD4 gene or the splicing variants having the sequences as described above, their naturally occurring mutant variants may also be used.

As described in the following examples, the present inventors have found out that the expression of SP3 protein is enhanced in the affected tissues of keloid patients, compared to that in the normal skin tissues of healthy person. Moreover, it was confirmed that the activities of IL-6 and NF-κB promoters are increased by the forced expression of SP3, and that the expression and activation of IL-6 and NF-κB which activate the inflammation amplifier are suppressed by specifically inhibiting the SP3 expression.

The inflammation amplifier is a molecular mechanism that induces chronic inflammation conditions reported by the present inventors. When an inflammatory stimulus is applied to type I collagen- positive non-immune cells such as fibroblasts, keratinocytes, and vascular endothelial cells, a NF-κB pathway and a STAT3 pathway are simultaneously activated in non-immune cells by cytokines such as IL-6 and IL-17 produced from the non-immune cells themselves and the induced T cells. Consequently, the production of cytokines such as IL-6, chemokines, and growth factors are synergistically increased. The produced IL-6 acts on the non-immune cells themselves, and activates the inflammation amplifier. Moreover, the chemokines and growth factors cause cell infiltration and dysregulates homeostasis. The fact that the continuous activation of inflammation amplifier induces chronic inflammatory conditions has been experimentally proved (Ogura et al., Immunity., 2008, 29(4), 628-36.; Murakami et al., J. Exp. Med., 2011, 208(1), 103-14.; Murakami et al., Cell Reports, 2013, 3(3), 946-59).

In this manner, the substance for suppressing the expression and/or inhibiting the activity of SP3 protein suppresses the activation of inflammation amplifier in the fibroblasts and keratinocytes constituting skin tissue, tendon, and tendon-surrounding tissue. Consequently, it is expected that the substance can be used as an active ingredient of a pharmaceutical for preventing and/or treating a chronic inflammatory disease that is induced by chronic inflammation.

The pharmaceutical in the first aspect of the present invention contains a substance for suppressing the expression and/or inhibiting the activity of SP3 protein as an active ingredient. "Contains as an active ingredient" means that the pharmaceutical contains an effective amount of the substance. The effective amount means an amount effective for preventing and/or treating a disease, and the effective amount is suitably determined according to the usage, the age of a target subject, the disease state, and other conditions.

The substance for suppressing the expression and/or inhibiting the activity of SP3 protein is a substance capable of suppressing the expression of mRNA of SP3 (may also be referred to as SP3 mRNA) from NEDD4 gene. The substance for suppressing the expression and/or inhibiting the activity of SP3 protein may be a substance capable of inhibiting selective splicing of mRNA transcribed from NEDD4 gene to SP3 mRNA, a substance capable of degrading mature SP3 mRNA after splicing, a substance capable of inhibiting protein translation from SP3 mRNA, or a substance capable of binding to SP3 protein and inhibiting the function.

A typical example of a substance for suppressing the expression of SP3 protein is an inhibitory nucleic acid such as antisense RNA or siRNA that can be designed and produced by those skilled in the art, on the basis of a known base sequence of NEDD4 gene or SP3 mRNA.

Moreover, the substance for suppressing the expression described in the present invention also includes shRNA containing the base sequence of siRNA; DNA capable of transcriptionally inducing the antisense RNA, siRNA, or the like described above, by being placed under the control of a suitable expression promoter; or RNA of which the stability against nuclease degradation is improved by modifying a part of the base sequence of the antisense RNA, siRNA, or the like described above, as a nucleic acid functionally equivalent to the antisense RNA or siRNA described above. The modifications to improve stability against degradation by nucleases include 2'-O-methylation, 2'-fluorination (F), and 4'-thiolation.

Furthermore, the substance for suppressing the expression described in the present invention also includes chimeric RNA in which a part of ribonucleotide of the RNA described above is replaced with a corresponding deoxyribonucleotide or a nucleotide analog. For example, the nucleotide analog includes 5-position modified uridine or cytidine such as 5-(2-amino) propyl uridine and 5-bromouridine; 8-position modified adenosine or guanosine such as 8-bromoguanosine; deazanucleotide such as 7-deazaadenosine; and O- or N-alkylated nucleotide such as N6-methyladenosine.

A typical example of a substance for inhibiting the activity of SP3 protein includes an anti-SP3 protein antibody that can specifically bind to SP3 protein to neutralize the activity, and a substance that inhibits a ubiquitin ligase.

The specific antibody described above used in the first aspect is an antibody that binds to SP3 protein, but that binds weakly or not at all to other proteins. The specific antibody may also be referred to as an antibody having a high binding affinity to SP3 protein. For example, the antibody having a high binding affinity to SP3 protein is an antibody with a dissociation constant K_{D} of 10⁻⁷ M or less, preferably 10⁻⁸ M or less, and more preferably 10⁻⁹ M or less.

The specific antibody is not limited by the animal species from which the antibody is derived. Moreover, the specific antibody may be any one of a monoclonal antibody, a polyclonal antibody, a human antibody, a chimeric antibody, and a humanized antibody. In addition, the specific antibody may also be an antibody consisting of full-length immunoglobulin, or may be an antibody derivative such as an antibody fragment composed of a part of an antibody. The antibody fragment includes F(ab')₂, Fab, diabody, Fv, ScFv, and Sc(Fv)₂ and the like.

The specific antibody can be prepared by a general antibody production method including a polyclonal antibody production method and a monoclonal antibody production method. In the polyclonal antibody production method, for example, a recombinant human SP3 protein preferably produced by genetic recombination techniques or the fragment thereof, such as a peptide containing amino acids at positions 431 to 455 of SP3 protein, is used as an antigen to immunize suitable laboratory animals such as rabbits, mice, and rats. In the monoclonal antibody production method, hybridomas from B cells derived from immunized animals and myeloma cells, or an antibody phage library is used.

The pharmaceutical in the first aspect may also contain a substance capable of suppressing the expression and/or inhibiting the activity of SP3 protein as an active ingredient, other than the substance described above. For example, such a substance may be obtained by screening the ability to suppress SP3 mRNA expression from a recombinant NEDD4 gene, or by screening the ability to inhibit the activity of SP3 protein using a recombinant SP3 protein.

As described in the following examples, by inhibiting the SP3 expression, the expression and activation of IL-6 and NF-κB that activate the inflammation amplifier are suppressed. Consequently, the present invention also provides an anti-inflammatory agent, or an expression inhibitor or an activation inhibitor against IL-6 or NF-κB, that contains a substance for suppressing the expression and/or inhibiting the activity of SP3 protein, as an active ingredient.

The pharmaceutical in the first aspect can be used by forming or formulating a pharmaceutical composition with drugs other than the active ingredient described above, or with pharmaceutically acceptable components such as buffers, antioxidants, preservatives, proteins, hydrophilic polymers, amino acids, chelating agents, nonionic surfactants, fillers, stabilizing agents, and carriers. The pharmaceutically acceptable components are known to those skilled in the art. Those skilled in the art can appropriately select and use the components from those described in the revised Japanese Pharmacopoeia, 17th edition or other standards, depending on dosage forms, within the scope of their normal implementation ability.

The chronic inflammatory disease that develops in skin, tendon, or tendon-surrounding tissue, and that can be prevented and/or treated with the pharmaceutical in the first aspect or a pharmaceutical composition containing thereof, may be a chronic inflammatory disease that develops in skin, tendon, or tendon-surrounding tissue, in which the expression of SP3 protein or SP3 mRNA is enhanced. Examples of such diseases include keloid, hypertrophic scar, autoimmune blistering disease, scleroderma, atopic dermatitis, contact dermatitis, eczema, psoriasis, pressure ulcer, bullous pemphigoid, alopecia, and Dupuytren's contracture. The chronic inflammatory disease to be prevented and/or treated is preferably keloid, hypertrophic scar, psoriasis, atopic dermatitis, bullous pemphigoid, scleroderma, or Dupuytren's contracture, and particularly preferably keloid.

The pharmaceutical in the first aspect, or the pharmaceutical composition containing thereof can be applied to the skin, tendon, or tendon-surrounding tissue of an affected area, or a site considered to be affected (for example, a wound site in keloid prevention) by any route as long as the effective amount can be delivered. A topical administration to the site is preferable. Moreover, for example, the formulation of the pharmaceutical or the pharmaceutical composition is preferably a formulation suitable for topical administration to skin, tendon, or tendon-surrounding tissue such as ointment, poultice, or injection.

The pharmaceutical in the first aspect can alleviate or ameliorate the symptoms, or prevent the onset of a chronic inflammatory diseasea, when administered to a target subject who needs treatment for the chronic inflammatory disease, or who may develop the disease. In other words, the first aspect of the present invention may also be referred to as a method for preventing and/or treating a chronic inflammatory disease in skin, tendon, or tendon-surrounding tissue by administering an effective amount of a substance for suppressing the expression and/or inhibiting the activity of SP3 protein to a target subject who needs treatment for the chronic inflammatory disease, or who may develop the disease. The effective amount of the pharmaceutical, formulation including the pharmaceutical, modes, administration method, and the like in the prevention and/or treatment method are as described in the pharmaceutical that is one aspect of the present invention.

Another aspect of the present invention relates to a method for determining susceptibility of a subject to a chronic inflammatory disease that develops in skin, tendon, or tendon-surrounding tissue, including a step of measuring the expression level of SP3 protein or SP3 mRNA in a sample collected from a subject; a step of comparing the measured expression level with the expression level of SP3 protein or SP3 mRNA in a sample collected from a healthy person; and a step of determining that the subject is susceptible to the chronic inflammatory disease, when the expression level in the sample from the subject is greater than that in the sample from the healthy person. The present aspect may also be referred to as a method for collecting data used for determining susceptibility of a subject to a chronic inflammatory disease, or a method for supplementing the susceptibility determination, including the three steps described above.

In addition to the fact that the expression of SP3 protein is enhanced in the affected skin tissues of keloid patients, the present inventors have found out that the SP3 mRNA expression is also enhanced in keratinocytes that are isolated from the tissues and that had undergone primary culture. The fact that the SP3 expression is also enhanced in keratinocytes become independent from the mass environment indicates that the SP3 expression is enhanced in the skin tissues of keloid patients, independent from the event of keloid development. It is assumed that the patient has developed keloid when the inflammatory stimulus from the wound has triggered the progression of inflammation amplifier, and SP3 has activated the inflammation amplifier and induced chronic inflammatory state. Consequently, data on the SP3 expression levels in a skin sample collected from a subject may serve as data for determining whether the subject is likely to develop keloid, in other words, data for determining susceptibility of the subject to keloid. Moreover, since SP3 is a positive controlling factor on the inflammation amplifier, it is considered that the subject with enhanced SP3 expression is susceptible to a chronic inflammatory disease other than keloid. Thus, data of SP3 expression level in a sample collected from a subject may also serve as data for determining susceptibility of the subject to a chronic inflammatory disease.

The sample may be skin, tendon, or tendon-surrounding tissue collected from a subject, or cells isolated therefrom. Alternatively, the sample may also be cell extract, total RNA, or the like extracted therefrom.

The SP3 expression level in the collected sample may be measured by a known method that can quantitatively measure the SP3 mRNA expression level, or a known method that can quantitatively measure SP3 protein.

Examples of the known method that can quantitatively measure the SP3 mRNA expression level include a PCR method using a set of primers composed of suitable base sequences designed on the basis of the base sequence of SP3 mRNA; a hybridization method using a probe that can specifically hybridize to SP3 mRNA, or DNA or RNA (cDNA or cRNA) complementary thereto; a microarray method using a chip on which the probe described above is fixed; and an RNA sequencing method. The SP3 mRNA expression level is preferably measured by a quantitative RT-PCR method, and typically by a real-time RT-PCR method.

In the quantitative RT-PCR method, the SP3 mRNA expression level is measured by synthesizing cDNA from SP3 mRNA using a reverse transcription reaction, and by using a primer set that specifically amplifies the cDNA. Any primer set can be used as long as the primer set anneals to cDNA that is reverse transcribed from SP3 mRNA, and that can specifically amplify the cDNA or a part thereof. Conditions such as a cDNA annealing site and the length of amplified region are not limited, and those skilled in the art can suitably select these conditions. In this example, the primer set can "Specifically amplify" in this context means that the primer set can amplify cDNA that is reverse transcribed from SP3 mRNA, but weakly or does not amplify cDNA complementary to other mRNAs including other NEDD4 splicing variants. It is preferable to design at least one primer sequence in the primer set so that the primer can selectively anneal to cDNA that is reverse transcribed from SP3 mRNA. Moreover, the length of primer is not particularly limited, and is typically, 15 to 30 bases.

In the real-time RT-PCR method using TaqMan (registered trademark) assay, a labeled probe that can specifically hybridize to the amplified region is used, in addition to the primer set. In the TaqMan (registered trademark) assay, amplicons are detected on the basis of the specific hybridization of probe. Thus, the primer set used in this method may preferentially amplify cDNA that is reverse transcribed from SP3 mRNA, over other template DNAs, and need not specifically amplify cDNA.

In a preferred embodiment using the real-time RT-PCR method using TaqMan (registered trademark) assay, an example of the primer set is a combination of primers composed of base sequences represented by SEQ ID No. 11 and 12, and an example of the probe is a base sequence represented by SEQ ID No. 10.

In the hybridization method and microarray method, the SP3 mRNA expression level is measured using a probe that can specifically hybridize to SP3 mRNA, or cDNA or cRNA complementary thereto. Any probe may be used as long as the probe can specifically hybridize to SP3 mRNA, or cDNA or cRNA complementary thereto. Conditions such as a hybridized position and the length of hybridizing region are not limited, and those skilled in the art can suitably select these conditions. Moreover, as long as the probe can specifically hybridize to SP3 mRNA, or cDNA or cRNA complementary thereto, the probe may contain one or more substitutions, deletions, or additions in the base sequence.

"Specifically hybridize" in this context means that the probe can hybridize to SP3 mRNA, or cDNA or cRNA complementary thereto, but hybridizes weakly or not at all to other mRNAs containing other NEDD4 splicing variants, or cDNA or cRNA complementary thereto. It is preferable to design the probe sequence on the basis of the base sequence only present in SP3 mRNA, or cDNA or cRNA complementary thereto. Moreover, the length of probe is not particularly limited, and for example, may be 10 to 500 bases.

The probe may also be a nucleic acid that can hybridize to SP3 mRNA, or cDNA or cRNA complementary thereto, under stringent conditions. Those skilled in the art may suitably design the stringent conditions by taking into account various factors that may affect the stringency such as temperature, probe concentration, probe length, reaction time, ionic strength, and salt concentration, and design the probe that can hybridize under stringent conditions. For example, the stringent conditions include 5 × SSC, 5 × Denhardt's solution, 0.5% SDS, 50% formamide, and 50°C.

Those skilled in the art can suitably design the primer set and probe, on the basis of the principle of the measuring method to be used, and the base sequence of SP3 mRNA, or cDNA or cRNA complementary thereto. The primer set and probe can then be prepared by a general nucleic acid synthesis method or a genetic engineering method. Moreover, according to the principle of the measuring method to be used, the primer set and probe may be labeled with a labeling compound such as fluorescent substances (for example, Cy3, Cy5, FITC, and Alexa Flour), radioisotopes (for example, ³H, ¹⁴C, ³²P, ³⁵S, ¹²⁵I, and ¹³¹I), enzymes, biotin, and streptavidin.

Examples of known methods that can quantitatively measure the expression level of SP3 protein include a quantitative immunoassay such as an enzyme-linked immunosorbent assay (ELISA) method, a radioimmunoassay (RIA) method, an in situ hybridization method, and an immunoblot method using an antibody that specifically binds to SP3 protein.

The antibody that specifically binds to SP3 protein is an antibody that binds to SP3 protein but that binds weakly or not at all to other proteins, and may also be referred to as an antibody having a high binding affinity to SP3 protein. For example, the antibody having a high binding affinity to SP3 protein is an antibody with a dissociation constant K_{D} of 10⁻⁷ M or less, preferably 10⁻⁸ M or less, and more preferably 10⁻⁹ M or less.

The specific antibody is not limited by the animal species from which the antibody is derived. Moreover, the specific antibody may also be any one of a monoclonal antibody, a polyclonal antibody, a human antibody, a chimeric antibody, and a humanized antibody. In addition, the specific antibody may also be an antibody consisting of full-length immunoglobulin, or may be an antibody derivative such as an antibody fragment composed of a part of an antibody. The antibody fragment includes F(ab')₂, Fab, diabody, Fv, ScFv, and Sc(Fv)₂ and the like.

The specific antibody can be prepared by a general antibody production method including a polyclonal antibody production method and a monoclonal antibody production method. In the polyclonal antibody production method, for example, a recombinant human SP3 protein preferably produced by genetic recombination techniques or the fragment thereof, such as a peptide containing amino acids at positions 431 to 455 of SP3 protein, is used as an antigen to immunize suitable laboratory animals such as rabbits, mice, and rats. In the monoclonal antibody production method, hybridomas from B cells derived from immunized animals and myeloma cells, or an antibody phage library is used.

The specific antibody may also be labeled with a labeling compound such as fluorescent substances (for example, FITC, rhodamine, phalloidin, and fluorescent protein), colloidal particles such as gold, fluorescent microbeads such as Luminex (registered trademark, Luminex Corporation), heavy metals (for example, gold and platinum), chromoproteins (for example, phycoerythrin and phycocyanin), radioisotopes (for example, ³H, ¹⁴C, ³²P, ³⁵S, ¹²⁵I, and ¹³¹I), enzymes (for example, peroxidase and alkaline phosphatase), biotin, and streptavidin.

The SP3 expression level in the sample collected from a subject is compared with the SP3 expression level in the sample collected from a healthy person, and when the expression level in the sample from the subject is greater than that in the sample from the healthy person, it is possible to determine that the subject is susceptible to a chronic inflammatory disease that develops in skin, tendon, or tendon-surrounding tissue. In this context, the healthy person is a person who is not suffering from the chronic inflammatory disease or a person who is less likely to suffer from the chronic inflammatory disease. The data on the SP3 expression level in the sample collected from the healthy person may be measured simultaneously with the sample collected from the subject, or may be prepared in advance.

The chronic inflammatory disease that develops in skin, tendon, or tendon-surrounding tissue, the susceptibility to which is to be determined, is as described above. Examples of such diseases include keloid, hypertrophic scar, autoimmune blistering disease, scleroderma, atopic dermatitis, contact dermatitis, eczema, psoriasis, pressure ulcer, bullous pemphigoid, alopecia, and Dupuytren's contracture. The chronic inflammatory disease is preferably keloid, hypertrophic scar, psoriasis, atopic dermatitis, bullous pemphigoid, scleroderma, or Dupuytren's contracture, and particularly preferably keloid.

In addition, when the SP3 expression level in the sample from the subject is greater than that in the sample from the healthy person, it not only means that the subject is susceptible to a chronic inflammatory disease, but also means that the subject is suitable for treatment with a pharmaceutical that contains a substance for suppressing the expression and/or inhibiting the activity of SP3 protein as an active ingredient. Consequently, another aspect of the present invention further includes a method for determining suitability for treatment with a pharmaceutical for a chronic inflammatory disease that develops in skin, tendon, or tendon-surrounding tissue, the pharmaceutical containing: a substance for suppressing the expression and/or inhibiting the activity of SP3 protein as an active ingredient, including a step of measuring the expression level of SP3 protein or SP3 mRNA in a sample collected from a subject; a step of comparing the measured expression level with the expression level of SP3 protein or SP3 mRNA in the sample collected from a healthy person; and a step of determining that the treatment with the pharmaceutical described above is suitable for the chronic inflammatory disease in the subject, when the expression level in the sample from the subject is greater than that in the sample from the healthy person. The meanings of terms in the determination method of the present aspect are the same as those in the susceptibility determination method. Moreover, the present aspect may also be referred to as a method for collecting data used for determining suitability for treatment with the pharmaceutical described above for a chronic inflammatory disease, or a method for supplementing the suitability determination, including the three steps described above.

For example, when the SP3 expression level in the sample from a subject who has not developed chronic inflammatory disease in skin, tendon, or tendon-surrounding tissue is greater than that in the sample from a healthy person, it is assumed that the pharmaceutical described above is effective for the subject as a prophylactic treatment against the chronic inflammatory disease. Moreover, when the SP3 expression level in the sample from a subject who has already developed chronic inflammatory disease in skin, tendon, or tendon-surrounding tissue is greater than that in the sample from a healthy person, it is assumed that the pharmaceutical described above is effective for the subject as a therapeutic treatment against the chronic inflammatory disease. Thus, the determination method in the present aspect determines that treatment for a chronic inflammatory disease with the pharmaceutical described above is suitable, when the pharmaceutical described above is assumed to be effective against the chronic inflammatory disease, and is useful as a companion diagnostic method for the use of the pharmaceutical described above.

The determination method in the present aspect may be performed in combination with the administration of pharmaceutical in the first aspect. In other words, an aspect of the present invention also includes a method for determining susceptibility of a subject to a chronic inflammatory disease that develops in skin, tendon, or tendon-surrounding tissue, and preventing and/or treating the chronic inflammatory disease, including a step of measuring the expression level of SP3 protein or SP3 mRNA in a sample collected from a subject; a step of comparing the measured expression level with the expression level of SP3 protein or SP3 mRNA in a sample collected from a healthy person; a step of determining that the subject is susceptible to the chronic inflammatory disease, when the expression level in the sample from the subject is greater than that in the sample from the healthy person; and a step of administering an effective amount of a substance for suppressing the expression and/or inhibiting the activity of SP3 protein to the subject who is determined to be susceptible to the chronic inflammatory disease.

Still another aspect of the present invention provides a pharmaceutical for preventing and/or treating a chronic inflammatory disease, that contains a substance for suppressing the expression and/or inhibiting the activity of NEDD4 protein expression as an active ingredient.

The present inventors have confirmed that the symptoms of arthritis and dermatitis are improved by administering an inhibitory nucleic acid capable of inhibiting NEDD4 expression, in an experimental system in which arthritis is developed by administering inflammatory cytokines such as IL-6 to model animals that spontaneously develop a chronic inflammatory arthritis similar to rheumatoid arthritis, and an experimental system in which dermatitis is developed by applying imiquimod to the animal skin. Consequently, it is expected that the substance for suppressing the expression and/or inhibiting the activity of NEDD4 protein can be used as an active ingredient of the pharmaceutical for preventing and/or treating a chronic inflammatory disease including arthritis and dermatitis.

The substance for suppressing the expression and/or inhibiting the activity of NEDD4 protein includes a substance capable of suppressing the mRNA expression from NEDD4 gene, a substance capable of inhibiting protein translation from the mRNA, or a substance capable of binding to NEDD4 protein and inhibiting the function. In this context, the NEDD4 protein is a part or all six NEDD4 protein splicing variants.

A typical example of a substance for suppressing the NEDD4 protein expression includes an inhibitory nucleic acid such as antisense RNA or siRNA that can be designed and produced by those skilled in the art, on the basis of a known base sequence of NEDD4 gene. Similar to the inhibitory nucleic acid in the pharmaceutical of the first aspect, the inhibitory nucleic acid that can be used in the present aspect includes a nucleic acid and chimeric RNA functionally equivalent to antisense RNA or siRNA. The substance for suppressing the NEDD4 protein expression may recognize the base sequence common to mRNAs of NEDD4 protein spicing variants, and may breakdown the mRNAs or inhibit the translation thereof.

A typical example of a substance for inhibiting the activity of NEDD4 protein includes an anti-NEDD4 antibody that can specifically bind to NEDD4 protein to neutralize the activity, a substance that inhibits a ubiquitin ligase, and the like.

The antibody that specifically binds to NEDD4 protein is an antibody that binds to NEDD4 protein but that binds weakly or not at all to other proteins, and may also be referred to as an antibody having a high binding affinity to NEDD4 protein. For example, the antibody having a high binding affinity to NEDD4 protein is an antibody with a dissociation constant K_{D} of 10⁻⁷ M or less, preferably 10⁻⁸ M or less, and more preferably 10⁻⁹ M or less.

The specific antibody is not limited by the animal species from which the antibody is derived. Moreover, the specific antibody may be any one of a monoclonal antibody, a polyclonal antibody, a human antibody, a chimeric antibody, and a humanized antibody. In addition, the specific antibody may also be an antibody consisting of full-length immunoglobulin, or may be an antibody fragment composed of a part of an antibody. The antibody fragment includes F(ab')₂, Fab, diabody, Fv, ScFv, and Sc(Fv)₂.

The specific antibody may be prepared by a general antibody production method including a polyclonal antibody production method and a monoclonal antibody production method. In the polyclonal antibody production method, for example, a recombinant human NEDD4 protein preferably produced by genetic recombination techniques or the fragment thereof, such as a peptide containing an amino acid sequence common in NEDD4 protein splicing variants, is used as an antigen to immunize suitable laboratory animals such as rabbits, mice, and rats. In the monoclonal antibody production method, hybridomas from B cells derived from immunized animals and myeloma cells, or an antibody phage library is used.

The substance for inhibiting the activity of NEDD4 protein may be an antibody that recognizes the amino acid sequence common in NEDD4 protein splicing variants, and that specifically binds to the amino acid sequence and neutralizes the activity.

The pharmaceutical in the present aspect may also contain a substance for suppressing the expression and/or inhibiting the activity of NEDD4 protein as an active ingredient, other than the substance described above. For example, such a substance may be obtained by screening the ability to suppress mRNA expression from a recombinant NEDD4 gene, or by screening the ability to inhibit the activity of NEDD4 protein using a recombinant NEDD4 protein.

The effective amount of the pharmaceutical and other composition components in the present aspect are the same as those of the pharmaceutical described in the first aspect.

The chronic inflammatory disease that can be prevented and/or treated with the pharmaceutical in the present aspect or the pharmaceutical composition containing thereof includes rheumatoid arthritis, systemic lupus erythematosus, Behcet's disease, Sjogren's syndrome, polymyositis, dermatomyositis, hyperthyroidism, hypothyroidism, autoimmune adrenal insufficiency, true red cell anemia, multiple sclerosis, autoimmune hepatitis, chronic hepatitis, hepatic cirrhosis, chronic obstructive pulmonary disease, Crohn's disease, and ulcerative colitis, in addition to the chronic inflammatory disease that develops in skin, tendon, or tendon-surrounding tissue described above. The chronic inflammatory disease to be prevented and/or treated is preferably rheumatoid arthritis. Moreover, in a specific embodiment, the chronic inflammatory disease to be prevented and/or treated with the pharmaceutical in the present aspect is preferably chronic inflammatory disease excluding keloid, chronic inflammatory disease excluding keloid and hypertrophic scar, or chronic inflammatory disease excluding chronic inflammatory skin disease.

The pharmaceutical in the present aspect or the pharmaceutical composition containing thereof may be administered by any route, as long as the effective amount can be delivered to the affected area, or a site considered to be affected. The pharmaceutical or the pharmaceutical composition containing thereof is preferably administered parenterally, such as intramuscularly, intravenously, intra-arterially, intraperitoneally, subcutaneously, and topically. Moreover, for example, the formulation of the pharmaceutical or pharmaceutical composition is preferably a formulation suitable for the administration route, such as injection, ointment, poultice, or drops.

The pharmaceutical in the present aspect can alleviate or ameliorate the symptoms, or prevent the onset of a chronic inflammatory disease, when administered to a target subject who needs treatment for the chronic inflammatory disease or who may develop the disease. In other words, still another aspect of the present invention may also be referred to as a method for preventing and/or treating a chronic inflammatory disease, by administering an effective amount of a substance for suppressing the expression and/or inhibiting the activity of NEDD4 protein to a target subject who needs treatment for the chronic inflammatory disease or who may develop the disease. The effective amount of the pharmaceutical, the formulation including the pharmaceutical, modes, administration method, and the like in the prevention and/or treatment method are as described above.

Still another aspect of the present invention relates to a method for determining susceptibility of a subject to a chronic inflammatory disease, including a step of detecting, in the sample collected from a subject rs8032158 that is a gene polymorphism of the base at position 26 in the base sequence represented by SEQ ID No. 43, present in the genomic sequence encoding an NEDD4 gene; and a step of determining that the subject is susceptible to the chronic inflammatory disease, when the genotype of the gene polymorphism described above is C/C or C/T. The present aspect may also be referred to as a method for collecting data used for determining susceptibility of a subject to a chronic inflammatory disease, or a method for supplementing the susceptibility determination, including the two steps described above.

Still another aspect of the present invention relates to a method for determining suitability for treatment with a pharmaceutical for a chronic inflammatory disease, the pharmaceutical containing: a substance for suppressing the expression and/or inhibiting the activity of SP3 protein as an active ingredient, including a step of detecting, in the sample collected from a subject, rs8032158 that is a gene polymorphism of the base at position 26 in the base sequence represented by SEQ ID No.43, present in the genomic sequence encoding an NEDD4 gene; and a step of determining that the treatment with the pharmaceutical described above is suitable for the chronic inflammatory disease in the subject, when the genotype of the gene polymorphism described above is C/C or C/T. The present aspect may also be referred to as a method for collecting data used for determining suitability for treatment with the pharmaceutical described above for a chronic inflammatory disease, or a method for supplementing the suitability determination, including the two steps described above.

The gene polymorphism rs8032158 is one of the single-nucleotide polymorphisms (SNPs) in NEDD4 gene. The gene polymorphism rs8032158 is an SNP in which the base at position 96068 in the base sequence of NEDD4
gene, registered in NCBI (https://www.ncbi.nlm.nih.gov/) as NG_051072.1, is changed from thymine (T) to cytosine (C) in the complementary strand (in NEDD4 gene, because the reverse strand in chromosomal DNA 15 is a sense strand, adenine (A) is at position 96068 in NG_051072.1). rs8032158 is located at position 55902679 in chromosomal 15. The relevance between the SNP and keloid development in Japanese patients with keloids has been reported (Nakashima et al., Nat. Genet., 2010, 42, 768-771).

The present inventors have found out that the SNP is present not only in keloid patients but also in patients with other chronic inflammatory disease more frequently than in healthy people. It is assumed that the SNP relates to the onset of a chronic inflammatory disease other than keloid, and it is expected that susceptibility to a chronic inflammatory disease can be determined on the basis of the SNP. Moreover, when the role of NEDD4 in the living body for activating the inflammation amplifier and inducing chronic inflammatory conditions is taken into account, the finding that the SNP is also present in the chronic inflammatory disease other than keloid, suggests that the pharmaceutical in the above described aspect that contains a substance for suppressing the expression and/or inhibiting the activity of NEDD4 protein or SP3 protein, which is a splicing variant of NEDD4 protein, as active ingredient, can successfully treat these chronic inflammatory diseases.

The SNP can be detected by using genomic DNA prepared from the subject. For example, the genomic DNA can be extracted, purified, and prepared according to a routine procedure, from any biological sample collected from a subject including body fluids such as blood, saliva, lymph fluid, airway mucus, bone-marrow aspirate, urine, semen, and peritoneal fluid; and tissue cells obtained by biopsy and the like.

The SNP is detected according to a conventional method known to those skilled in the art. Examples of such a method include, but not limited to, a method using amplified fragments containing the SNP amplified by PCR such as a nucleic acid sequence-based amplification (NASBA) method, a ligase chain reaction (LCR) method, a strand displacement amplification (SDA) method, a loop-mediated isothermal amplification (LAMP) method, and TaqMan (registered trademark) PCR; and a method in which the base sequence including the SNP is directly determined by using a DNA sequencer and the like. The method also includes a hybridization method in which a probe specific for the SNP is used; a detecting method using a microarray such as a DNA chip, a Gene chip, a microchip, and a bead array; a method using chemical cleavage of mismatches (CCM); a primer extension (PEX) method; and an invader method.

An example of the method using amplified fragments containing the SNP amplified by PCR is a method using a primer set designed such that amplified fragments are generated only when the target SNP is contained, and that one of a sense primer and an antisense primer hybridizes to the SNP. By using such a primer set, it is possible to detect the target SNP by finding out whether the amplified fragments are generated.

Another method using amplified fragments is a method using a primer set designed such that a region containing the target SNP is amplified. The method using such a primer set can detect the SNP on the basis of a difference between the sizes of the amplified fragments, base sequences, conformations, the presence of a restriction site, and the like. For example, it is possible to detect the target SNP from a difference between the mobility of the amplified fragments, when agarose gel electrophoresis, polyacrylamide gel electrophoresis, capillary electrophoresis, or the like is used.

Detection of the SNP by hybridization can be performed using a probe specific to the SNP. Any probe can be used as long as the probe can specifically hybridize to the base sequence in genomic DNA containing the target SNP. For example, the probe may be oligonucleotide that can hybridize to the genomic DNA base sequence containing the target SNP of at least 10 consecutive bases or more, preferably 10 to 100 consecutive bases, and more preferably 10 to 50 consecutive bases. Moreover, it is preferable to design the probe sequence such that the base corresponding to the target SNP is located near the center of the sequence.

As long as the probe is specific to the target SNP, in other words, as long as the probe hybridizes to the genomic DNA base sequence containing the target SNP of at least 10 consecutive bases or more, but does not hybridize to other sequences, the probe may contain one or more substitutions, deletions, or additions. The probe may also be labeled, when necessary, with suitable means such as a fluorescent substance or radioactive substance.

Moreover, the probe may also be oligonucleotide that can hybridize to the genomic DNA base sequence containing the target SNP of at least 10 consecutive bases or more, under stringent conditions. The stringent conditions are as described above.

When the detected SNP is homotype or heterotype, in other words, when the genotype of the SNP is C/C or C/T, particularly C/C, it is possible to determine that the subject is susceptible to a chronic inflammatory disease. The chronic inflammatory disease the susceptibility to which is to be determined, is preferably a chronic inflammatory disease that develops in skin, tendon, or tendon-surrounding tissue such as keloid, hypertrophic scar, autoimmune blistering disease, scleroderma, atopic dermatitis, contact dermatitis, eczema, psoriasis, pressure ulcer, bullous pemphigoid, alopecia, and Dupuytren's contracture. For example, the preferable chronic inflammatory disease the susceptibility to which is to be determined is chronic inflammatory diseases excluding keloid, such as hypertrophic scar, psoriasis, atopic dermatitis, bullous pemphigoid, scleroderma, pressure ulcer, alopecia, or Dupuytren's contracture; and particularly, hypertrophic scar, scleroderma, atopic dermatitis, bullous pemphigoid, scleroderma, or Dupuytren's contracture.

Another aspect of the present invention relates to a kit for determining susceptibility to a chronic inflammatory disease that develops in skin, tendon, or tendon-surrounding tissue; the kit described above includes a primer set or a probe that detects SP3 mRNA expression, or a specific antibody against SP3 protein.

Still another aspect of the present invention relates to a kit for determining suitability for treatment with a pharmaceutical for a chronic inflammatory disease that develops in skin, tendon, or tendon-surrounding tissue; the kit described above includes a primer set or a probe that detects SP3 mRNA expression, or a specific antibody against SP3 protein, wherein the pharmaceutical is a pharmaceutical that contains a substance for suppressing the expression and/or inhibiting the activity of SP3 protein as an active ingredient.

Still another aspect of the present invention relates to a kit for determining susceptibility to a chronic inflammatory disease (excluding keloid); the kit described above includes a primer set or a probe that detects rs8032158 that is a genetic polymorphism of the base at position 26 in the base sequence represented by SEQ ID No. 43, present in the genomic sequence encoding an NEDD4 gene.

Still another aspect of the present invention relates to a kit for determining suitability for treatment with a pharmaceutical for a chronic inflammatory disease; the kit includes a primer set or a probe that detects rs8032158 that is a genetic polymorphism of the base at position 26 in the base sequence represented by SEQ ID No. 43, present in the genomic sequence encoding an NEDD4 gene, wherein the pharmaceutical contains a substance for suppressing the expression and/or inhibiting the activity of SP3 protein as an active ingredient.

The primer set, probe, and specific antibody included in the kit described above, and their usage are as described above. Moreover, for example, in addition to the primer set, probe, or specific antibody, the kit described above may also include dNTP, an enzyme, a labeling or detecting reagent, a control reagent (for example, positive or negative control oligonucleotide or antigen), a buffer solution, a pH regulator, a stabilizer, a solid phase support, a reaction vessel, and an instruction manual, according to each measuring or detecting method.

The kit described above including the primer set or probe may also include an internal control primer set or probe for detecting a housekeeping gene such as GAPDH and actin. Moreover, the kit described above including the specific antibody may further include a secondary antibody conjugated with a labelling substance such as a fluorescent substance and horseradish peroxidase for detecting the specific antibody bound to SP3 protein, and an antibody that binds to SP3 protein at a site different from that of the specific antibody described above.

The present invention will now be described in more detail with reference to the following examples. However, the present invention is not limited to these examples.

### Examples

### Example 1. Expression Analysis of SP3 in Keloid Patients

### 1) Sample Collection

The study protocol was approved by the medical ethics committee of Hokkaido University Graduate School of Medicine and was conducted in accordance with the Declaration of Helsinki principles. Written informed consents were obtained from all patients. 14 samples of masses (7 from ear pinna, 5 from truncus, 1 from shoulder, and 1 from neck) were collected from keloid patients. 5 samples of normal skins (4 from truncus and 1 from shoulder) were collected from non-keloid patients. These samples were used in the following experiments.

### 2) Expression Analysis of Splicing Variant Using Real-time PCR

Keratinocytes and fibroblasts were isolated from masses and normal skin tissues (N = 5 to 7 each) according to a routine procedure. The keratinocytes were primarily cultured in KGM-Gold (trademark) keratinocyte growth medium (Clonetics, Lonza). The fibroblasts were primarily cultured in Dulbecco's Modified Eagle's Medium (DMEM; Life Technologies) added with 10% fetal bovine serum. By using a spin column method (ISOSPIN Cell & Tissue, DNase I (RNase free) Set, NIPPON GENE), total RNA was prepared from primary culture cells. cDNA was prepared from total RNA, by performing reverse transcription using M-MLV Reverse Transcriptase (Promega).

Real-time PCRs were performed on cDNA by the TaqMan probe method with KAPA PROBE (registered trademark) FAST qPCR Master Mix (2x) kit ABI Prism (KAPA BIOSYSTEMS), and the primers and probes listed in Table 1. The expression levels of SP1 to SP5 and SP7 that are NEDD4 splicing variants were analyzed by a calibration curve method in which GAPDH is the internal control.

**Table 1**

| **Gene** | **Primer sequence (5' to 3')** | | **SEQ ID** |
|---|---|---|---|
| **NM_7007.6 (GAPDH)** | Probe | GATGCCAGCCCCAGCGTCAAAGGT | SEQ ID No.1 |
| | Forward | CTCCTCTGACTTCAACAGCGA | SEQ ID No.2 |
| | Reverse | CCAAATTCGTTGTCATACCAGGA | SEQ ID No.3 |
| **NM_006154 (SP1)** | Probe | CGTCACTCTCACGTAAGGATCACTAGCTCC | SEQ ID No.4 |
| | Forward | ATAGGCCTTGCCAAGAAGGATA | SEQ ID No.5 |
| | Reverse | ACTTTGGATTCAAACTCTTTTTAATGG | SEQ ID No.6 |
| **NM_198400 (SP2)** | Probe | GTCTAATTCTTGCAAGCCTGGCTGGGTTGT | SEQ ID No.7 |
| | Forward | GCATCTGGAAATGAAGGTAAAGTGG | SEQ ID No.8 |
| | Reverse | TGGCAAGCAGCATCTGGTTG | SEQ ID No.9 |
| **NM_001284338 (SP3)** | Probe | ATCCATTACCGACAGAAAATCCAAGATTGGAGAGA | SEQ ID No.10 |
| | Forward | GGATAATTTATCAAGAGACAGCAACA | SEQ ID No.11 |
| | Reverse | CAAAACAACCCAGCCAGGCT | SEQ ID No12 |
| **NM_001284339 (SP4)** | Probe | ATATCCTGCCTCTCTTCCCACCCTGGAG | SEQ ID No.13 |
| | Forward | TGAGGAATTAGAGCAACAACAAGAA | SEQ ID No.14 |
| | Reverse | CATGGTTTACATAATAGGTCCTTCCA | SEQ ID No.15 |
| **NM_001284340 (SP5)** | Probe | ACAGCAACAGAGATTGCACAAATGAACTGT | SEQ ID No.16 |
| | Forward | TCTGGAAATGAAGGTAAAGTGGATAA | SEQ ID No.17 |
| | Reverse | CTCCAATCTTGGATTTTCTGTCTTG | SEQ ID No.18 |
| **NM_001329212 (SP7)** | Probe | CGTCACTCTCACGTAAGGATCACTAGCTCC | SEQ ID No.19 |
| | Forward | ATAGGCCTTGCCAAGAAGGATA | SEQ ID No.20 |
| | Reverse | AGTATAACTTTTTAATGGTTTTTGTTTGC | SEQ ID No.21 |

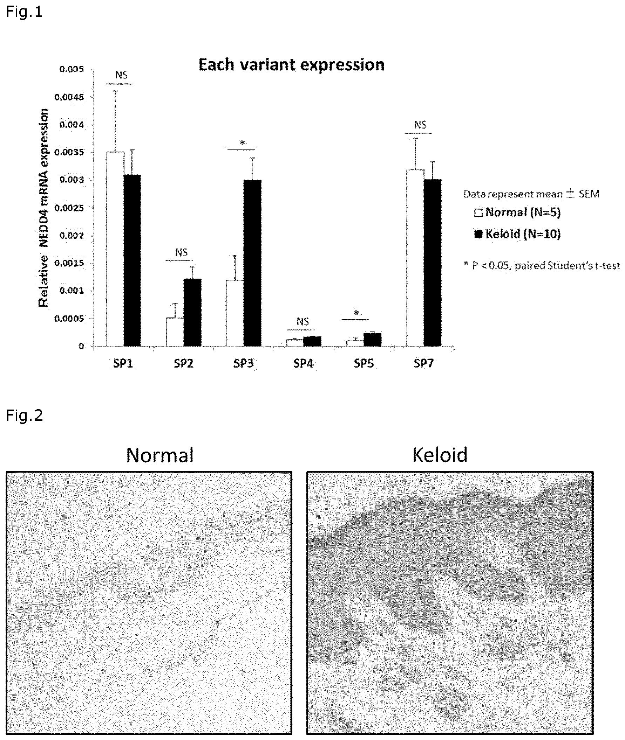

Fig. 1 illustrates quantitative results in keratinocytes. It was confirmed that SP3 mRNA expression is enhanced in keloids, compared to that in normal skins, and that there is no significant difference in the expression levels of the splicing variants other than SP3, between the normal skin tissues and keloid tissues. The same tendency was also identified in the fibroblasts.

### 3) Expression Analysis of SP3 Protein in Skin Tissue Using Immune Tissue Staining

An SP3 identifying antibody (SP3₄₃₁₋₄₅₅ specific antibody) was produced using a peptide having an amino acid sequence corresponding to NEDD4 exon 6 that is not present in NEDD4 splicing variants 1 and 7 (CQININGELERPHSQMNKNHGILRR (SEQ ID No. 22), corresponding to positions 431 to 455 in NEDD4-SP3 amino acid) (Cosmo Bio Co., Ltd.). By using SP3 identifying antibody as a primary antibody, and biotinylated anti-rabbit IgG monoclonal antibody (Vector) as a secondary antibody, immunohistological staining was performed on sections of masses from keloid patients and normal skin from non-keloid patients.

Fig. 2 illustrates representative immunohistological staining images. It was confirmed that the expression of SP3 protein was enhanced both in epidermis and dermis in keloid skin tissues, compared to those in normal skin tissues.

### Example 2. Activation of Inflammation Amplifier by SP1 or SP3

DNA corresponding to NEDD4 SP1 registered as NM_006154 or DNA corresponding to NEDD4 SP3 registered as NM_001284338 in NCBI was incorporated into a pEF-BOS vector (Nucleic Acids Res. 18, 5322, 1990), and an NEDD4 SP1 or NEDD4 SP3 expression vector was constructed. The NEDD4 SP1 or NEDD4 SP3 expression vector was transfected into two types of HK293T cells that were transformed by pGL4.32 [luc2P/ IL-6-RE/ Hygro] (Promega) having a firefly luciferase gene under the IL-6 promoter, or pGL4.32 [luc2P/ NF-κB-RE/Hygro] (Promega) having a firefly luciferase gene under the NFκB promoter, and an expression vector pGL4.32 (Promega) of Renilla luciferase, which is an internal control. The transfected HEK293T cells were stimulated with TNFα (50 ng/ml), and the cells were harvested five hours after stimulation. A change in the promoter activity of the IL-6 promoter or NF-κB promoter in the presence or absence of TNFα stimulation was assessed by dual luciferase assay. The luciferase activity was measured by a luminometer (GloMax (registered trademark)-multi detection system, Promega) using a Dual-Luciferase assay system (Promega). The luminescence intensity by the firefly luciferase reaction was corrected with the luminescence intensity by the Renilla luciferase reaction.

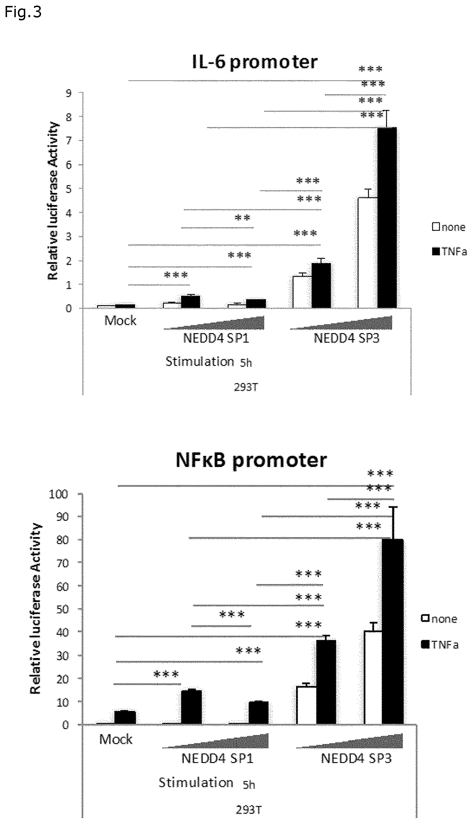

Fig. 3 illustrates the results of luciferase assay. The vertical axis indicates the corrected luminescence intensity. It was identified that the forced expression of SP1 and SP3 increases the IL-6 promoter activity and the NF-κB promoter activity. It was also identified that SP3 increases the promoter activity more than SP1.

### Example 3. Effects of SP3 Inhibitory Nucleic Acid on Inflammation Amplifier

### 1) Effects on IL-6 mRNA Expression

siRNA (GCAAGACAAGAGAUGAUUUtt (SEQ ID No. 23), Ambion Silencer Select siRNA, Thermo Fisher Scientific) against NEDD4 SP3, and control siRNA (Ambion Silencer Select siRNA, Thermo Fisher Scientific) were transfected into a human glioma cell line (H4 cells) using Lipofectamine RNAiMAX (Thermo Fisher Scientific), and NEDD4 SP3 knockdown cells were constructed. After stimulating the control cells and the knockdown cells with IL-6 + TNFα, the IL-6 mRNA expression was measured by qPCR (primer sequence: GGTACATCCTCGACGGCATCT (SEQ ID No. 24) and GTGCCTCTTTGCTGCTTTCAC (SEQ ID No.25)). Moreover, the knockdown efficiency was confirmed using the NEDD4 SP3 expression as an index. The expression level indicates the relative amount with respect to the housekeeping gene GAPDH (primer sequence: GAGTCAACGGATTTGGTCGT (SEQ ID No.26) and CGCTCCTGGAAGATGGTG (SEQ ID No.27)).

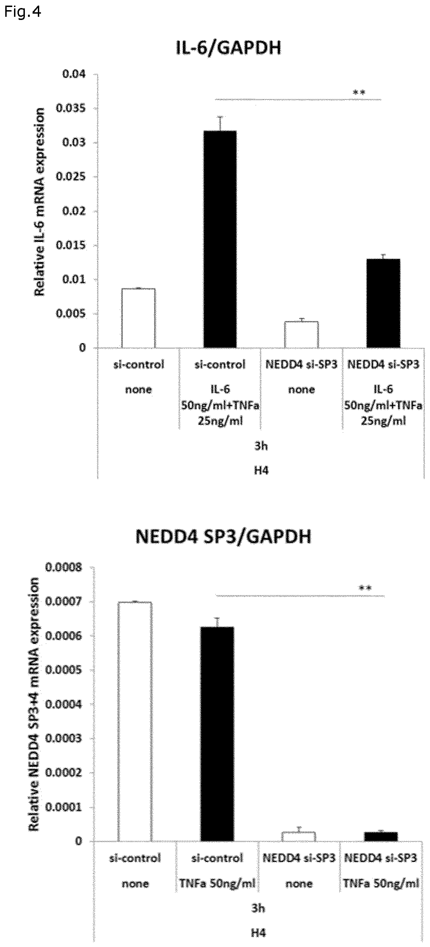

Fig. 4 illustrates the results. Compared to the H4 cells introduced with si-control, in the H4 cells introduced with NEDD4 si-SP3, NEDD4 SP3 mRNA expression level was reduced to 4.2% under TNF-α stimulation, and NEDD4 si-SP3 knockdown was confirmed. Compared to the H4 cells introduced with si-control, in the H4 cells introduced with NEDD4 si-SP3, IL-6 mRNA expression level was reduced to 41.0% under IL-6 and TNF-α stimulation. Consequently, it was indicated that NEDD4 si-SP3 suppresses the activation of the inflammation amplifier.

### 2) Effects of NF-κB on Phosphorylation

siRNA (GCAAGACAAGAGAUGAUUUtt (SEQ ID No.23), Ambion Silencer Select siRNA, Thermo Fisher Scientific) against NEDD4 SP3, and control siRNA (Ambion Silencer Select siRNA, Thermo Fisher Scientific) were transfected into a human glioma cell line (H4 cells) using Lipofectamine RNAiMAX (Thermo Fisher Scientific), and NEDD4 SP3 knockdown cells were constructed. The control cells and the NEDD4 SP3 knockdown cells were stimulated with TNFα (50 ng/ml). The cells were lysed at 0, 5, 15, and 30 minutes after stimulation, and all cellular fractions were harvested. To extract all cellular fractions, the cells were lysed using the high-salt cell lysis buffer (NaCl 600 mM). After sufficient vortexing, the samples were slowly mixed and stirred by inversion at 4°C for 30 minutes, and centrifuged at a high speed (15000 rpm) for 30 minutes. The supernatant from which cell residues have been removed, was harvested as all cellular fractions. At every process, 1/100 amount of protease inhibitor (Sigma Aldrich) was added to the cell lysis buffer. The obtained samples were all boiled in SDS sample buffer containing 2-mercaptoethanol at 95°C for five minutes, and polyacrylamide electrophoresis (SDS-PAGE) was performed on the samples with SDS-PAGE gel (Wako Pure Chemical Industries, Ltd.) having the concentration gradient of 5 to 12%, using a wet western blot system (Bio-Rad). The p65 phosphorylation of each sample was assessed by western blotting. The proteins were detected using Pierce ECL Western Blotting Substrate (Thermo Fisher), by using an anti-phosphorylated p65 (Ser 536) antibody (Cell Signaling Technology) and an anti-p65 antibody (Santa Cruz). An anti-tubulin antibody (Sigma-Aldrich) was also used as the internal control. A horseradish peroxidase labeling antibody (Southern Biotech) was used as the secondary antibody. The chemiluminescence was then exposed to a film using a film processor FPM 100 (Fuji Medical Film).

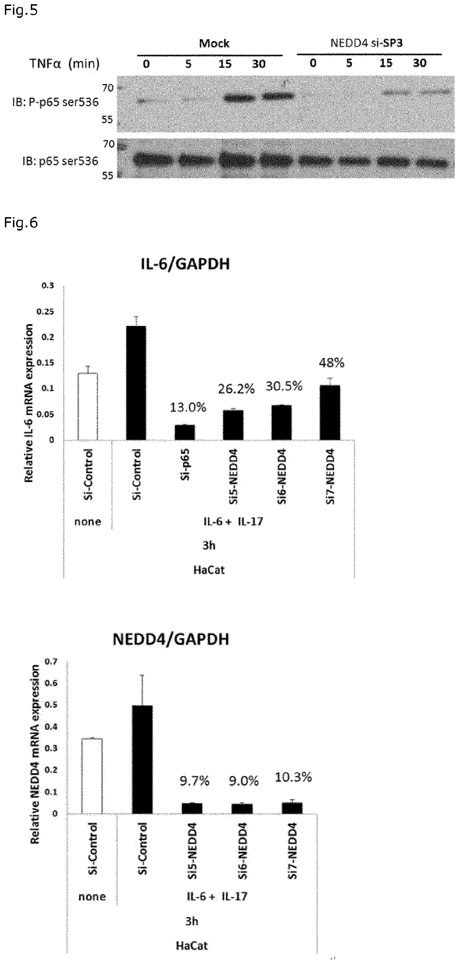

Fig. 5 illustrates the results. Compared to the H4 cells introduced with si-control, in the H4 cells introduced with NEDD4 si-SP3, phosphorylation of NF-κB p65 subunit at ser536 was suppressed. From this result also, it was indicated that NEDD4 si-SP3 suppresses the activation of the inflammation amplifier.

### Example 4. Effects of NEDD4 Inhibitory Nucleic Acid on Inflammatory Amplifier

NEDD4 siRNA (three types of siRNAs that knock down all variants: (si5: GAUCACCUCUCUUACUUCAt (SEQ ID No.28), si6: GGAAGAUCCAAGAUUGAAAt (SEQ ID No.29), and si7: GGCGAUUUGUAAACCGAAUtt (SEQ ID No.30), Ambion Silencer Select siRNA, Thermo Fisher Scientific), or control siRNA (Ambion Silencer Select siRNA, Thermo Fisher Scientific) was transfected into human keratinocyte cell line (HaCaT cells) or glioma cell line (H4 cells) using Lipofectamine RNAiMAX (Thermo Fisher Scientific), and NEDD4 knockdown cells were constructed. After stimulating the control cells and NEDD4 knockdown cells with IL-6 + IL-17, IL-6 mRNA expression was measured by qPCR (primer sequence: GGTACATCCTCGACGGCATCT (SEQ ID No.24) and GTGCCTCTTTGCTGCTTTCAC (SEQ ID No.25)). Moreover, the knockdown efficiency was confirmed using the expression of NEDD4 (primer sequence: ATGGCAAACTGTTGGATGGT (SEQ ID No.31) and GGTCCAATTCTGTTGGGTCA (SEQ ID No.32)) as an index. The expression level indicates the relative amount with respect to the housekeeping gene GAPDH (primer sequence: GAGTCAACGGATTTGGTCGT (SEQ ID No.26) and CGCTCCTGGAAGATGGTG (SEQ ID No.27)).

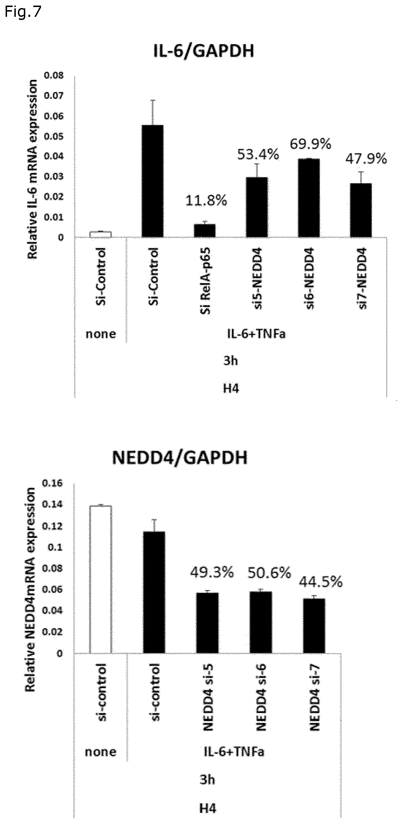

Fig. 6 illustrates the results in HaCat cells, and Fig. 7 illustrates the results in H4 cells. In both cells, the three types of NEDD4 siRNAs suppress the NEDD4 mRNA expression enhancement respectively, and suppresses the IL-6 mRNA expression enhancement, under IL-6 + IL-17 stimulation. Consequently, it was indicated that NEDD4 siRNA suppresses the activation of the inflammation amplifier.

### Example 5. Effects of NEDD4 Inhibitory Nucleic Acid in Rheumatoid Arthritis Model Animals

As a human rheumatoid arthritis model, F759 mice developing a chronic inflammatory arthritis (Atsumi et al., J. Exp. Med. 2002, 196(7), 979-90.) were used. On test days 0, 2, and 4, 3.6 × 10⁵ TU/dose of a lentivirus (NEDD4 sh-1-RNA: TRCN0000092433 (SEQ ID No.33); NEDD4 sh-2-RNA: TRCN0000092435 (SEQ ID No.34), Sigma Aldrich) that expresses shRNA for all NEDD4 splicing variants was dissolved in PBS, and was administered to the hindlimb joints of eight-week F759 mice (n=3) through injection. Then, on test days 5, 6, and 7, 100 ng of human IL-6 (Toray) and 100 ng of mouse IL-17A (Biolegend) were dissolved in PBS, and was administered to the hindlimb joints of mice to induce arthritis. The control group (NTC) was the mice (n=3) administered only with PBS through injection, instead of the lentivirus. The symptoms of arthritis were assessed with clinical scores for 30 days from the start of test. The clinical score determination was performed according to the previous report (J Exp Med. 2011 Jan 17; 208(1): 103-14.).

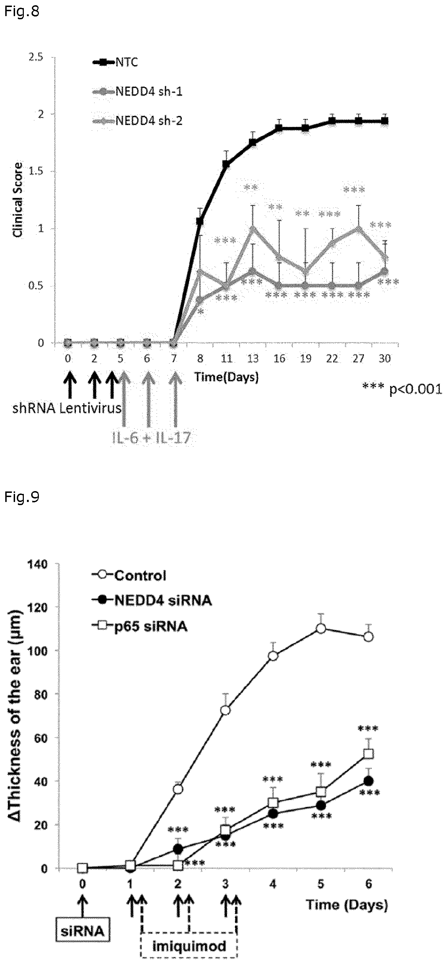

Fig. 8 illustrates the change of clinical scores. By administering shRNA that knocks down NEDD4 gene expression to the hindlimb joints through injection, it was possible to suppress the progression of arthritis induced by IL-6 and IL-17.

### Example 6. Effects of NEDD4 Inhibitory Nucleic Acid in Dermatitis Model Animals

According to the reports (Kanemaru et al., Exp Dermatol 2015; 24(6): 436-42.; Morimura et al., J Dermatol Sci 2016; 82(3): 175-88.), a siRNA mixture (containing 2.8 ul of cream-based ointment (Johnson & Johnson) and 4.7 ul of 20 uM siRNA) was applied to the ear pinna of C57BL/6 mice (test days 0, 1, 2 and 3). As the siRNA, control siRNA (Accell Non-targeting siRNA #1 from Dharmacon Inc.), siRNAs (SMARTpool-Accell Nedd4 siRNA from Dharmacon Inc.; SEQ ID No.35 to 38) that knock down all NEDD4 splicing variants; or p65 siRNA (SMARTpool-Accell Rela (p65) siRNA from Dharmacon Inc.; SEQ ID No.39 to 42) that knocks down NF-κB p65 subunit expression was used. On test days 1, 2, and 3, 2.5 ul of Toll-like receptor 7 agonist imiquimod (Beselna cream 5%, Mochida Pharmaceutical) was applied to the same ear pinna, to induce skin inflammation. The degree of skin inflammation was assessed by measuring the thickness of the ear with a micro-caliper.

Fig. 9 illustrates the change of ear thickness. Similar to the siRNA that knocks down NF-κB p65 subunit expression, the siRNA that knocks down NEDD4 gene expression could suppress the progression of dermatitis induced by imiquimod.

### Example 7. SNP Analysis of NEDD4 Gene in Chronic Inflammatory Skin Disease Patients

### 1) Collection of Samples

In addition to the mass samples from keloid patients and the normal skin samples from non-keloid patients used in Example 1, skin samples from affected areas were collected from psoriatic patients (n=5), patients with hypertrophic scar (n=4), and scleroderma patients (n=3), and used in the following experiments.

### 2) SNP Analysis on NEDD4 Gene

A part of isolated skin tissues was resected, immediately placed in PAXgene (registered trademark) Tissue Container (QIAGEN), and was made to react at 4°C overnight. Then, the skin tissues were extracted, placed in PAXgene Tissue Stabilizer (QIAGEN), and were stored at minus 20°C until further use. RNA and genomic DNA were extracted from a part (equivalent to 10 mg) of the tissues stored in PAXgene Tissue Stabilizer using AllPrep DNA/RNA Mini kit (QIAGEN). To confirm rs8032158 SNP (base indicated by [C/T] in the sequence TTGGGAAAAAACTAGCTTATAATAA[C/T]ATAGGTAGTTTATAACATTTATTAA (SEQ ID No.43), https://www.ncbi.nlm.nih.gov/snp/?term=rs8032158) present in the intron region of NEDD4 gene, PCR direct sequencing was performed on the extracted genomic DNA. The sequence of the used primer is CGACTGCATGGTGGCTTTAT (SEQ ID No.44) and TCCTGACTAGCGGCGATTAC (SEQ ID No.45). The PCR amplifications were performed using the following cycle conditions: 35 cycles of a 10-second denaturation step at 98°C, a 30-second annealing step at 60°C, and a 30-second cDNA replication reaction step at 72°C. Each PCR product was purified with Wizard (registered trademark) SV Gel and PCR Clean-UP System (Promega). The sequencing reaction (primer sequence: CGACTGCATGGTGGCTTTAT (SEQ ID No.44) was performed on 10 ng of the purified PCR product, using BigDye (registered trademark) Terminator v3.1 Cycle Sequencing Kit (Applied Biosystems). The sequencing reaction was performed using the following cycle conditions: 25 cycles of a 10-second denaturation step at 96°C, a 5-second annealing step at 50°C, and a 4-minute cDNA replication reaction step at 60°C. The sequencing reaction products were then purified by ethanol precipitation, and sequencing analysis was performed using an ABI PRISM 3130 Genetic Analyzer (Applied Biosystems).

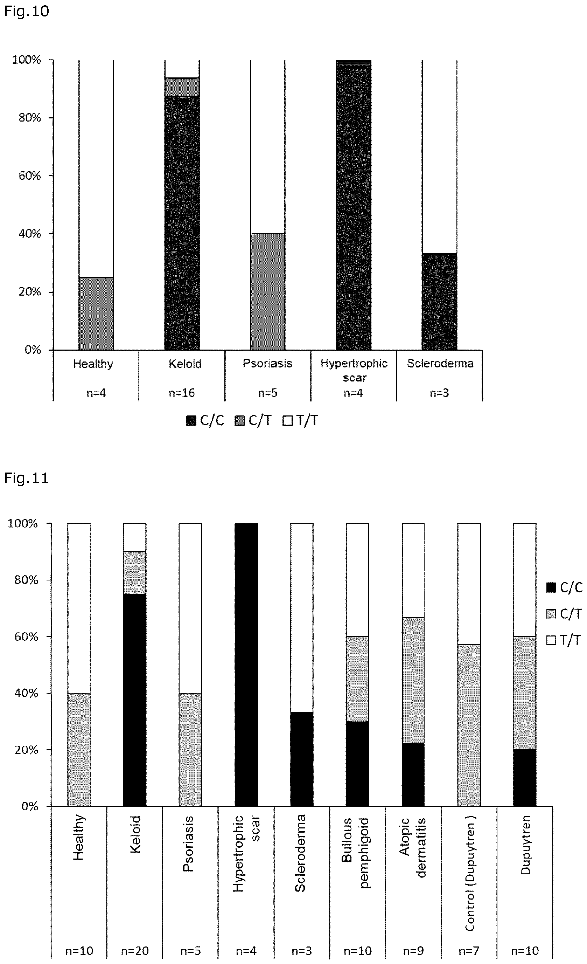

Fig. 10 illustrates the SNP frequency of NEDD4 genes. As previously reported, in the samples derived from keloid patients, T was changed to C, and in particular, Homotype C/C was frequently identified. C/C was also identified in the samples derived from patients with hypertrophic scar, and in the samples derived from scleroderma patients.

### Example 8. SNP Analysis on NEDD4 Genes in Patients with Chronic Inflammatory Skin Disease and Disease in Tendon-Surrounding Tissue

By using the following skin samples, SNP analysis was performed on NEDD4 genes using the same technique as that in Example 6.

Normal Skin Samples from Control: Non-keloid patients (healthy people; n=10), carpal tunnel syndrome patients (Control (Dupuytren); n=7)

Skin Samples from Patients' Affected Areas: keloid (n=20), psoriasis (n=5), hypertrophic scar (n=4), scleroderma (n=3), bullous pemphigoid (n=10), atopic dermatitis (n=9), Dupuytren's contracture (n=10)

Fig. 11 illustrates the SNP frequency of NEDD4 genes. In addition to from keloid, hypertrophic scar, and scleroderma confirmed in Example 7, Homotype C/C was also detected in the skin samples of patients with bullous pemphigoid, atopic dermatitis, and Dupuytren's contracture. The C/C gene type and high NEDD4 SP3 expression are in correlation with each other, and NEDD4 SP3 is increased in these diseases. Consequently, it is expected that a substance suppressing the expression of NEDD4 SP3 and a substance inhibiting the activity of NEDD4 SP3 can successfully treat these diseases.

## Claims

1. A pharmaceutical for preventing and/or treating a chronic inflammatory disease that develops in skin, tendon, or tendon-surrounding tissue, the pharmaceutical comprising:
a substance for suppressing expression and/or inhibiting activity of NEDD4 splicing variant 3 protein as an active ingredient.

2. The pharmaceutical according to claim 1, wherein the substance for suppressing the expression of the protein is an inhibitory nucleic acid that suppresses expression of NEDD4 splicing variant 3 mRNA or that inhibits translation of the protein from the mRNA.

3. The pharmaceutical according to claim 1, wherein the substance for inhibiting the activity of the protein is an antibody or a derivative thereof that specifically binds to the protein.

4. The pharmaceutical according to any one of claims 1 to 3, wherein the chronic inflammatory disease includes keloid, hypertrophic scar, psoriasis, atopic dermatitis, bullous pemphigoid, scleroderma, or Dupuytren's contracture.

5. A method for determining susceptibility of a subject to a chronic inflammatory disease that develops in skin, tendon, or tendon-surrounding tissue, the method comprising:
a step of measuring an expression level of NEDD4 splicing variant 3 protein or NEDD4 splicing variant 3 mRNA in a sample collected from a subject;
a step of comparing the measured expression level with an expression level of NEDD4 splicing variant 3 protein or NEDD4 splicing variant 3 mRNA in a sample collected from a healthy person; and
a step of determining that the subject is susceptible to the chronic inflammatory disease, when the expression level in the sample from the subject is greater than that in the sample from the healthy person.

6. The method according to claim 5, wherein the chronic inflammatory disease includes keloid, hypertrophic scar, psoriasis, atopic dermatitis, bullous pemphigoid, scleroderma, or Dupuytren's contracture.

7. A method for determining suitability for treatment with a pharmaceutical for a chronic inflammatory disease that develops in skin, tendon, or tendon-surrounding tissue, the pharmaceutical containing: a substance for suppressing expression and/or inhibiting activity of NEDD4 splicing variant 3 protein as an active ingredient, and the method comprising:
a step of measuring an expression level of NEDD4 splicing variant 3 protein or NEDD4 splicing variant 3 mRNA in a sample collected from a subject;
a step of comparing the measured expression level with an expression level of NEDD4 splicing variant 3 protein or NEDD4 splicing variant 3 mRNA in a sample collected from a healthy person; and
a step of determining that the treatment with the pharmaceutical is suitable for the chronic inflammatory disease in the subject, when the expression level in the sample from the subject is greater than that in the sample from the healthy person.

8. The method according to claim 7, wherein the chronic inflammatory disease includes keloid, hypertrophic scar, psoriasis, atopic dermatitis, bullous pemphigoid, scleroderma, or Dupuytren's contracture.

9. A pharmaceutical for preventing and/or treating a chronic inflammatory disease (excluding keloid), the pharmaceutical comprising:
a substance for suppressing expression and/or inhibiting activity of NEDD4 protein as an active ingredient.

10. The pharmaceutical according to claim 9, wherein the substance for suppressing the expression of the protein is an inhibitory nucleic acid that suppresses expression of mRNA encoding the protein or that inhibits translation of the protein from the mRNA.

11. The pharmaceutical according to claim 9, wherein the substance for inhibiting the activity of the protein is an antibody or a derivative thereof that specifically binds to the protein.

12. The pharmaceutical according to any one of claims 9 to 11, wherein the chronic inflammatory disease includes arthritis, hypertrophic scar, psoriasis, atopic dermatitis, bullous pemphigoid, scleroderma, or Dupuytren's contracture.

13. A method for determining susceptibility of a subject to a chronic inflammatory disease (excluding keloid), the method comprising:
a step of detecting, in a sample collected from a subject, rs8032158 that is a gene polymorphism of a base at position 26 in a base sequence represented by SEQ ID No. 43 present in a genomic sequence encoding an NEDD4 gene; and
a step of determining that the subject is susceptible to the chronic inflammatory disease (excluding keloid), when a genotype of the gene polymorphism is C/C or C/T.

14. The method according to claim 13, wherein the chronic inflammatory disease includes hypertrophic scar, psoriasis, atopic dermatitis, bullous pemphigoid, scleroderma, or Dupuytren's contracture.

15. A kit for determining susceptibility to a chronic inflammatory disease that develops in skin, tendon, or tendon-surrounding tissue, the kit comprising:
a primer set or a probe that detects expression of NEDD4 splicing variant 3 mRNA.

16. A kit for determining susceptibility to a chronic inflammatory disease that develops in skin, tendon, or tendon-surrounding tissue, the kit comprising:
a specific antibody against NEDD4 splicing variant 3 protein.

17. A kit for determining suitability for treatment with a pharmaceutical for a chronic inflammatory disease that develops in skin, tendon, or tendon-surrounding tissue, the kit comprising:
a primer set or a probe that detects expression of NEDD4 splicing variant 3 mRNA, wherein
the pharmaceutical contains a substance for suppressing expression and/or inhibiting activity of NEDD4 splicing variant 3 protein as an active ingredient.

18. A kit for determining suitability for treatment with a pharmaceutical for a chronic inflammatory disease that develops in skin, tendon, or tendon-surrounding tissue, the kit comprising:
a specific antibody against NEDD4 splicing variant 3 protein, wherein
the pharmaceutical contains a substance for suppressing expression and/or inhibiting activity of NEDD4 splicing variant 3 protein as an active ingredient.

19. A kit for determining susceptibility to a chronic inflammatory disease (excluding keloid), the kit comprising:
a primer set or a probe that detects rs8032158 that is a gene polymorphism of a base at position 26 in a base sequence represented by SEQ ID No. 43 present in a genomic sequence encoding an NEDD4 gene.


SEQUENCE LISTING

<110>  National University Corporation Hokkaido University

<120>  Pharmaceutical for chronic inflammatory disease and Method for
       determining susceptibility to chronic inflammatory disease

<130>  P17HU04WO

<160>  45

<170>  PatentIn version 3.5

<210>  1
<211>  24
<212>  DNA
<213>  Artificial Sequence

<220>
<223>  synthesized oligonucleotide

<400>  1
gatgccagcc ccagcgtcaa aggt                                              24


<210>  2
<211>  21
<212>  DNA
<213>  Artificial Sequence

<220>
<223>  synthesized oligonucleotide

<400>  2
ctcctctgac ttcaacagcg a                                                 21


<210>  3
<211>  23
<212>  DNA
<213>  Artificial Sequence

<220>
<223>  synthesized oligonucleotide

<400>  3
ccaaattcgt tgtcatacca gga                                               23


<210>  4
<211>  30
<212>  DNA
<213>  Artificial Sequence

<220>
<223>  synthesized oligonucleotide

<400>  4
cgtcactctc acgtaaggat cactagctcc                                        30


<210>  5
<211>  22

<212>  DNA
<213>  Artificial Sequence

<220>
<223>  synthesized oligonucleotide

<400>  5
ataggccttg ccaagaagga ta                                                22


<210>  6
<211>  27
<212>  DNA
<213>  Artificial Sequence

<220>
<223>  synthesized oligonucleotide

<400>  6
actttggatt caaactcttt ttaatgg                                           27


<210>  7
<211>  30
<212>  DNA
<213>  Artificial Sequence

<220>
<223>  synthesized oligonucleotide

<400>  7
gtctaattct tgcaagcctg gctgggttgt                                        30


<210>  8
<211>  25
<212>  DNA
<213>  Artificial Sequence

<220>
<223>  synthesized oligonucleotide

<400>  8
gcatctggaa atgaaggtaa agtgg                                             25


<210>  9
<211>  20
<212>  DNA
<213>  Artificial Sequence

<220>
<223>  synthesized oligonucleotide

<400>  9
tggcaagcag catctggttg                                                   20


<210>  10
<211>  35
<212>  DNA
<213>  Artificial Sequence

<220>
<223>  synthesized oligonucleotide

<400>  10
atccattacc gacagaaaat ccaagattgg agaga                                35


<210>  11
<211>  26
<212>  DNA
<213>  Artificial Sequence

<220>
<223>  synthesized oligonucleotide

<400>  11
ggataattta tcaagagaca gcaaca                                          26


<210>  12
<211>  20
<212>  DNA
<213>  Artificial Sequence

<220>
<223>  synthesized oligonucleotide

<400>  12
caaaacaacc cagccaggct                                                 20


<210>  13
<211>  28
<212>  DNA
<213>  Artificial Sequence

<220>
<223>  synthesized oligonucleotide

<400>  13
atatcctgcc tctcttccca ccctggag                                        28


<210>  14
<211>  25
<212>  DNA
<213>  Artificial Sequence

<220>
<223>  synthesized oligonucleotide

<400>  14
tgaggaatta gagcaacaac aagaa                                           25


<210>  15
<211>  26
<212>  DNA
<213>  Artificial Sequence

<220>
<223>  synthesized oligonucleotide

<400>  15
catggtttac ataataggtc cttcca                                            26


<210>  16
<211>  30
<212>  DNA
<213>  Artificial Sequence

<220>
<223>  synthesized oligonucleotide

<400>  16
acagcaacag agattgcaca aatgaactgt                                        30


<210>  17
<211>  26
<212>  DNA
<213>  Artificial Sequence

<220>
<223>  synthesized oligonucleotide

<400>  17
tctggaaatg aaggtaaagt ggataa                                            26


<210>  18
<211>  25
<212>  DNA
<213>  Artificial Sequence

<220>
<223>  synthesized oligonucleotide

<400>  18
ctccaatctt ggattttctg tcttg                                             25


<210>  19
<211>  30
<212>  DNA
<213>  Artificial Sequence

<220>
<223>  synthesized oligonucleotide

<400>  19
cgtcactctc acgtaaggat cactagctcc                                        30


<210>  20
<211>  22
<212>  DNA
<213>  Artificial Sequence

<220>
<223>  synthesized oligonucleotide

<400>  20
ataggccttg ccaagaagga ta                                                22

<210>  21
<211>  29
<212>  DNA
<213>  Artificial Sequence

<220>
<223>  synthesized oligonucleotide

<400>  21
agtataactt tttaatggtt tttgtttgc                                         29


<210>  22
<211>  25
<212>  PRT
<213>  Artificial Sequence

<220>
<223>  synthesized peptide

<400>  22

Cys Gln Ile Asn Ile Asn Gly Glu Leu Glu Arg Pro His Ser Gln Met
1               5                   10                  15


Asn Lys Asn His Gly Ile Leu Arg Arg
            20                  25


<210>  23
<211>  21
<212>  DNA
<213>  Artificial Sequence

<220>
<223>  synthesized oligonucleotide

<400>  23
gcaagacaag agaugauuut t                                                 21


<210>  24
<211>  21
<212>  DNA
<213>  Artificial Sequence

<220>
<223>  synthesized oligonucleotide

<400>  24
ggtacatcct cgacggcatc t                                                 21


<210>  25
<211>  21
<212>  DNA
<213>  Artificial Sequence

<220>
<223>  synthesized oligonucleotide

<400>  25
gtgcctcttt gctgctttca c                                                 21


<210>  26
<211>  20
<212>  DNA
<213>  Artificial Sequence

<220>
<223>  synthesized oligonucleotide

<400>  26
gagtcaacgg atttggtcgt                                                   20


<210>  27
<211>  18
<212>  DNA
<213>  Artificial Sequence

<220>
<223>  synthesized oligonucleotide

<400>  27
cgctcctgga agatggtg                                                     18


<210>  28
<211>  20
<212>  DNA
<213>  Artificial Sequence

<220>
<223>  synthesized oligonucleotide

<400>  28
gaucaccucu cuuacuucat                                                   20


<210>  29
<211>  20
<212>  DNA
<213>  Artificial Sequence

<220>
<223>  synthesized oligonucleotide

<400>  29
ggaagaucca agauugaaat                                                   20


<210>  30
<211>  21
<212>  DNA
<213>  Artificial Sequence

<220>
<223>  synthesized oligonucleotide

<400>  30
ggcgauuugu aaaccgaaut t                                                 21

<210>  31
<211>  20
<212>  DNA
<213>  Artificial Sequence

<220>
<223>  synthesized oligonucleotide

<400>  31
atggcaaact gttggatggt                                                    20


<210>  32
<211>  20
<212>  DNA
<213>  Artificial Sequence

<220>
<223>  synthesized oligonucleotide

<400>  32
ggtccaattc tgttgggtca                                                    20


<210>  33
<211>  58
<212>  DNA
<213>  Artificial Sequence

<220>
<223>  synthesized oligonucleotide

<400>  33
ccgggcgaca tacttagtac cacatctcga gatgtggtac taagtatgtc gctttttg          58


<210>  34
<211>  58
<212>  DNA
<213>  Artificial Sequence

<220>
<223>  synthesized oligonucleotide

<400>  34
ccgggctcaa gaagcagact gacatctcga gatgtcagtc tgcttcttga gctttttg          58


<210>  35
<211>  19
<212>  DNA
<213>  Artificial sequence

<220>
<223>  synthesized oligonucleotide

<400>  35
ccacuauguu gaucgguga                                                     19


<210>  36
<211>  19

<212>  DNA
<213>  Artificial sequence

<220>
<223>  synthesized oligonucleotide

<400>  36
gcauucuuuu cgaaguguu                                                    19


<210>  37
<211>  19
<212>  DNA
<213>  Artificial sequence

<220>
<223>  rs8032158 SNP reverse primer

<400>  37
cguuugacaa gagaugauu                                                    19


<210>  38
<211>  19
<212>  DNA
<213>  Artificial sequence

<220>
<223>  synthesized oligonucleotide

<400>  38
guugugagag ugaagguua                                                    19


<210>  39
<211>  19
<212>  DNA
<213>  Artificial Sequence

<220>
<223>  synthesized oligonucleotide

<400>  39
cucugaacua auaaauguc                                                    19


<210>  40
<211>  19
<212>  DNA
<213>  Artificial Sequence

<220>
<223>  synthesized oligonucleotide

<400>  40
gauuccgcua uaaaugcga                                                    19


<210>  41
<211>  19
<212>  DNA
<213>  Artificial Sequence

<220>
<223>  synthesized oligonucleotide

<400>  41
ccagugugug aagaagcga                                                    19


<210>  42
<211>  19
<212>  DNA
<213>  Artificial Sequence

<220>
<223>  synthesized oligonucleotide

<400>  42
ggauugaaga gaagcgcaa                                                    19


<210>  43
<211>  51
<212>  DNA
<213>  Homo sapiens

<400>  43
ttgggaaaaa actagcttat aataayatag gtagtttata acatttatta a               51


<210>  44
<211>  20
<212>  DNA
<213>  Artificial Sequence

<220>
<223>  synthesized oligonucleotide

<400>  44
cgactgcatg gtggctttat                                                   20


<210>  45
<211>  20
<212>  DNA
<213>  Artificial Sequence

<220>
<223>  synthesized oligonucleotide

<400>  45
tcctgactag cggcgattac                                                   20